# EUROPEAN PATENT APPLICATION

(11) **EP 4 640 685 A1**
(43) Date of publication of application: **29.10.2025**
(21) Application number: 22969243.9
(22) Date of filing: 23.12.2022
(51) Int. Cl.: C07D 471/04

(54) **PRODUCTION INTERMEDIATE**

(71) Applicant: Carna Biosciences, Inc., Kobe-shi, Hyogo 650-0047 (JP)
(72) Inventor: SEERDEN, Jean Paul, Kobe-shi, Hyogo 650-0047 (JP); DE JONG, Johannes, Kobe-shi, Hyogo 650-0047 (JP); SHIMIZU, Tomohiro, Kobe-shi, Hyogo 650-0047 (JP); IRIE, Takayuki, Kobe-shi, Hyogo 650-0047 (JP); SAWA, Masaaki, Kobe-shi, Hyogo 650-0047 (JP)
(74) Representative: Kador & Partner Part mbB
(86) International application number: PCT/JP2022/047526
(87) International publication number: WO 2024/134859

(57) **Abstract**

The following formula (Ia): 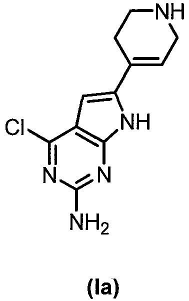 or a salt thereof provided by the present invention is useful as a production intermediate for producing the Compound (A) having a BTK inhibitory action.

## Description

### TECHNICAL FIELD

The present invention relates to a novel production intermediate in the production of an oxoisoquinoline derivative having a BTK inhibitory action and methods for producing the same.

### BACKGROUND ART

Compounds with Bruton's tyrosine kinase (BTK) inhibitory activity are useful for the treatment of diseases in which BTK signaling is involved, such as cancer, B-cell lymphoma, and chronic lymphocytic leukemia, and are also considered to be useful for the treatment of solid cancers in which p65BTK is expressed. They are also useful for the treatment of allergic diseases, autoimmune diseases, inflammatory diseases, and the like.

Oxoisoquinoline derivatives have been found to be especially useful as BTK inhibitors, and Patent Document 1 illustrates 2-(3-{2-amino-6-[1-(oxetan-3-yl)-1,2,3,6-tetrahydropyridin-4-yl]-7H-pyrrolo[2,3-d]pyrimidin-4-yl}-2-(hydroxymethyl)phenyl)-6-cyclopropyl-8-fluoroisoquinolin-1(2H)-one [hereinafter referred to as "Compound (A)"]:

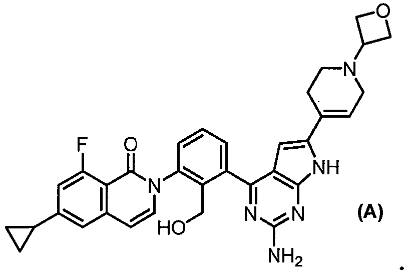

However, the method for producing the Compound (A) disclosed in Patent Document 1 is extremely difficult to carry out in a semi-industrial or industrial scale. It has been required to find a production method suitable for practical production by further improving the operability of the production, purification efficiency, yield, and the like.

### CITATION LIST

### PATENT DOCUMENT

Patent Document 1: WO 2018/097234 pamphlet

### SUMMARY OF THE INVENTION

### PROBLEMS TO BE SOLVED BY THE INVENTION

In order to produce a high-quality pharmaceutical compound in an industrial scale, it is necessary to develop a novel production intermediate that is suitable for practical production, as well as to solve problems such as reducing the number of column chromatography purification steps, improving the operability of the production process and yield, increasing purity, and reducing the use of harmful solvents or the like.

The present invention provides a novel production intermediate and production methods for producing an oxoisoquinoline derivatives more economically and in higher yields using said intermediate. Furthermore, the methods disclosed in the present description provide an economical cost-saving process for the production intermediate and a higher yield in total compared to the currently known production method (disclosed in Patent Document 1).

### MEANS TO SOLVE PROBLEMS

The methods for producing the Compound (A) provided by the present invention are as follows.
(1) A method for producing the Compound (A), wherein the Compound (Ia) is oxetanylated to produce the Compound (Ib), and then reacted with the known Compound (B) to produce the target Compound (A). 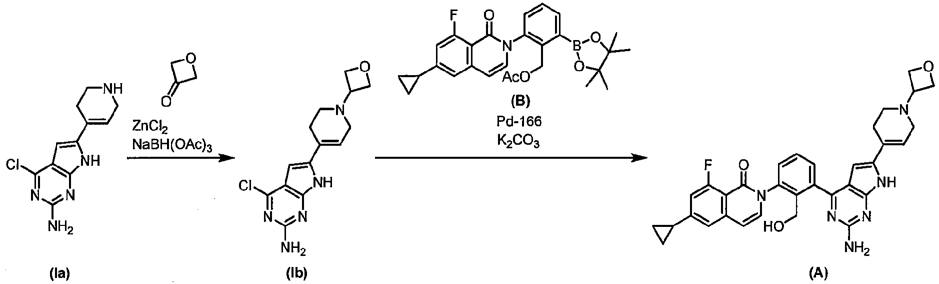
(2) A method for producing the Compound (A), wherein the Compound (III) is chlorinated, and then the trifluoroacetyl group is deprotected to produce the Compound (Ia) to be used in the above production method (1). 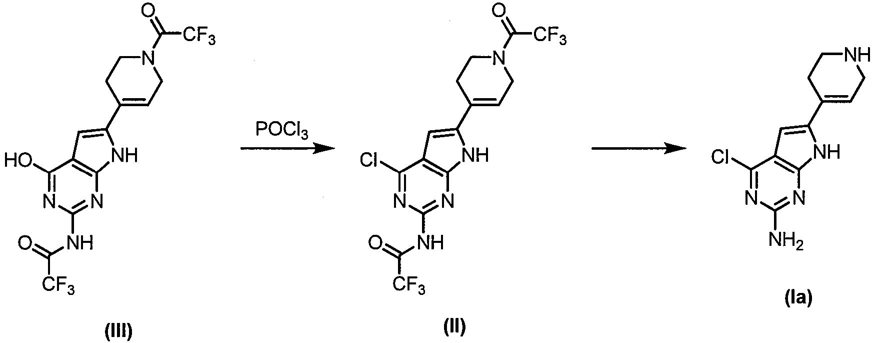
(3) A method for producing the Compound (A), wherein the Compound (IV) is treated with trifluoroacetic anhydride to produce the Compound (III) to be used in the above production method (2). 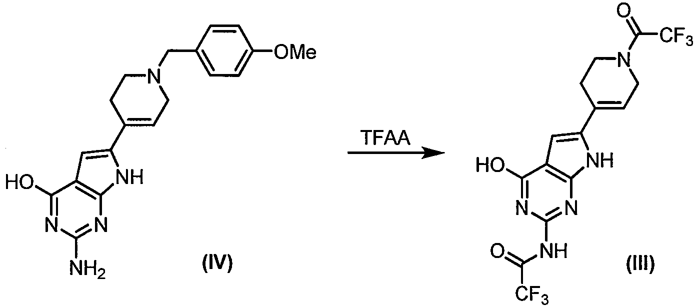
(4) A method for producing the Compound (A), wherein the Compound (V) is treated with sodium borohydride to produce the Compound (IV) to be used in the above production method (3). 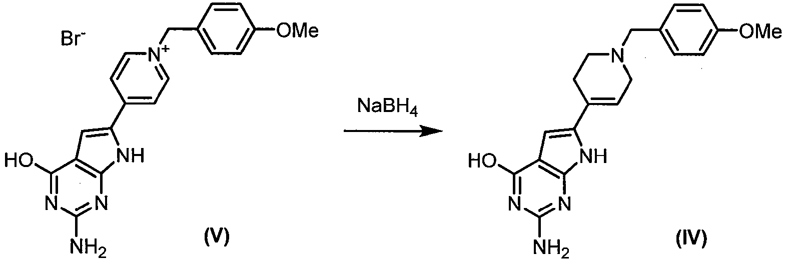
(5) A method for producing the Compound (A), wherein the Compound (VII) is reacted with a brominating agent, and then reacted with the Compound (VI) to produce the Compound (V) to be used in the above production method (4). 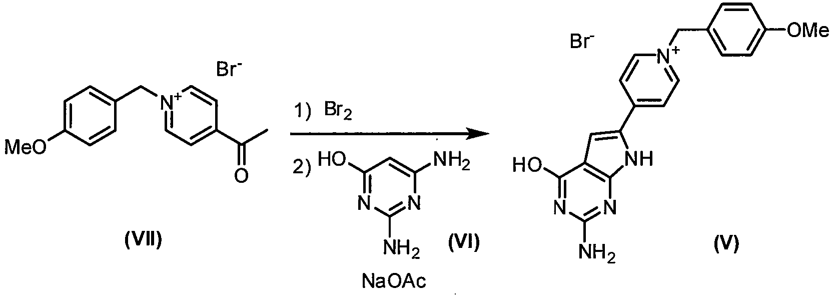 The present invention also provides the intermediate Compound (Ia) for producing the Compound (A) and methods for producing the same as follows.
(6) A compound represented by the following formula (Ia): 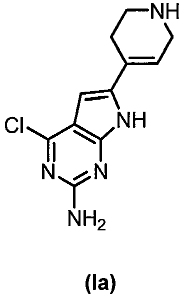 or a salt thereof.
(7) A method for producing the Compound (Ia), wherein the Compound (III) is chlorinated, and then treated with ammonia water to produce the Compound (Ia). 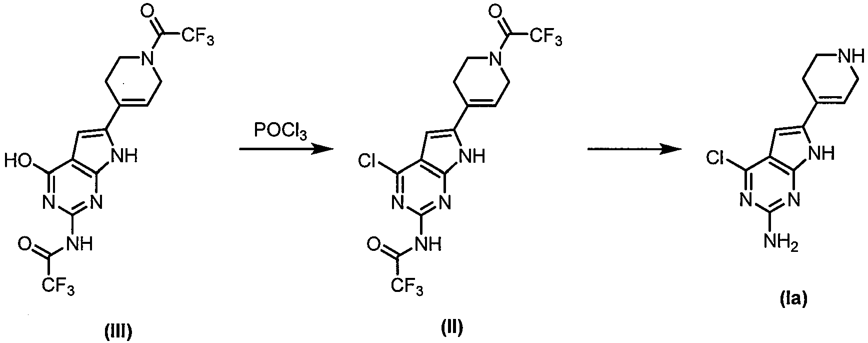
(8) A method for producing the Compound (Ia), wherein the Compound (IV) is treated with trifluoroacetic anhydride to produce the Compound (III) to be used in the above production method (7). 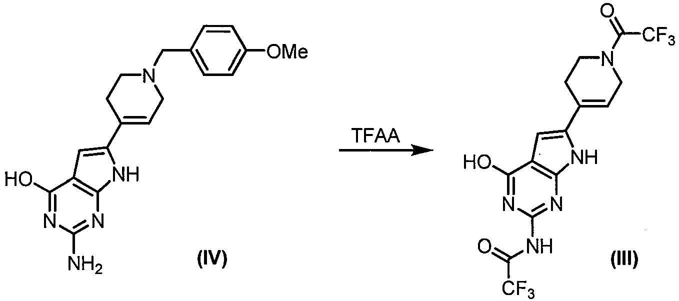
(9) A method for producing the Compound (Ia), wherein the Compound (V) is treated with sodium borohydride to produce the Compound (IV) to be used in the above production method (8). 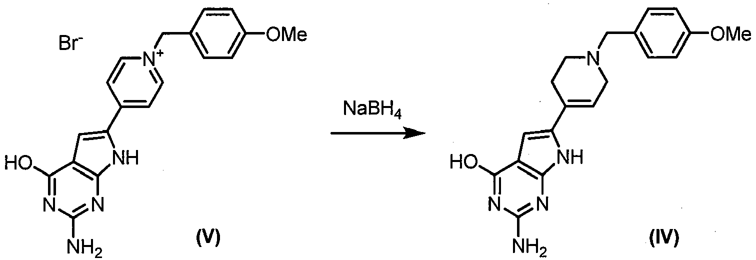
(10) A method for producing the Compound (Ia), wherein the Compound (VII) is reacted with a brominating agent, and then reacted with the Compound (VI) to produce the Compound (V) to be used in the above production method (9).

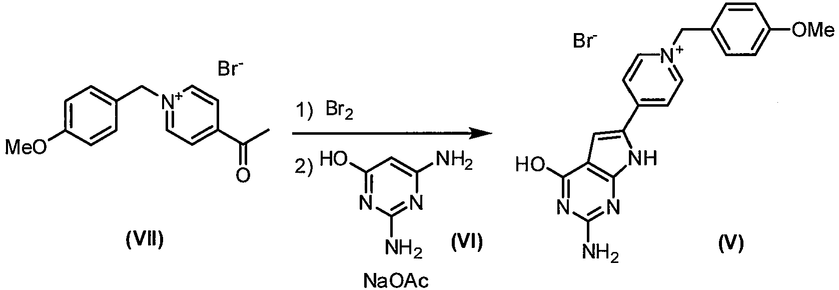

### EFFECT OF INVENTION

According to the production methods of the present invention using the novel production intermediate, the target Compound (A) can be produced more economically and in higher yields. Furthermore, the methods disclosed in the present description provide an economical cost-saving process for the production intermediate and a higher yield in total compared to the currently known production method (disclosed in Patent Document 1).

### MODE FOR CARRYING OUT THE INVENTION

The embodiments of the present invention are as follows.

The abbreviations and symbols used in the following explanations mean the followings.
Ac: acetyl group
DCM: dichloromethane
DMA: N,N-dimethylacetamide
DMSO: dimethyl sulfoxide
EtOH: ethanol
THF: tetrahydrofuran
NBS: N-bromosuccinimide
Pd-166: dichlorobis(tert-butyldicyclohexylphosphine) palladium(II)
TBME: tert-butyl methyl ether

(1) The Compound (Ia) is oxetanylated, and then reacted with the known Compound (B) to produce the target Compound (A).

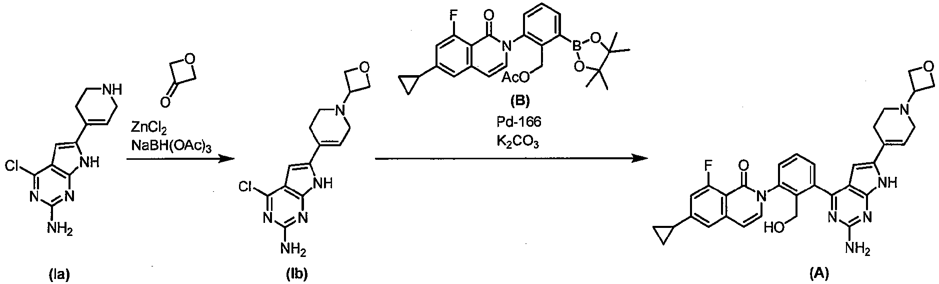

### (1-1) Production of Compound (Ib)

In DMF, to the Compound (Ia) are added 2 equivalents of 3-oxetanone and 0.1 equivalent of zinc chloride, and finally added 2.4 equivalents of sodium triacetoxyborohydride, and the resulting mixture is reacted. Then, ammonia water is added thereto to quench the reaction to give the precipitated Compound (Ib).

### (1-2) Production of Compound (A)

In a mixed solvent of DMSO-water, in the presence of 2 equivalents of potassium carbonate and 0.008 equivalent of Pd-166, the Compound (Ib) and the Compound (B) are reacted at 90 to 100°C for 1 to 2 hour(s). Then, water is added thereto, and the precipitated compound is collected by filtration to give the target Compound (A).

(2) The Compound (III) is chlorinated, then the trifluoroacetyl group is deprotected to produce the Compound (Ia), and then subjected to the above step (1) to produce the target Compound (A).

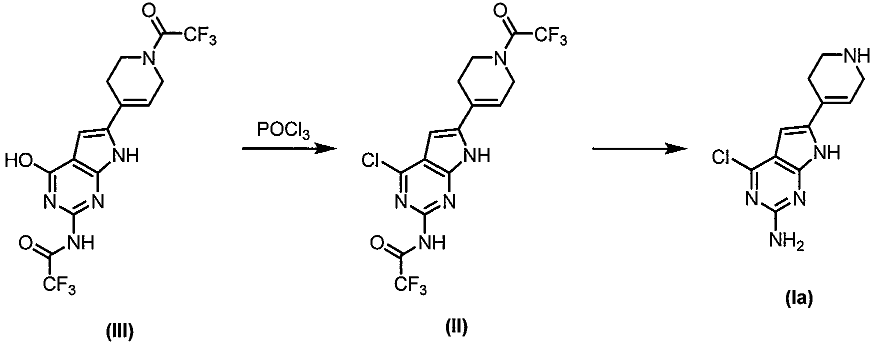

### (2-1) Production of Compound (Ia)

In acetonitrile, the Compound (III) is reacted with 4 to 8 equivalents, preferably 6 equivalents of phosphoryl chloride to produce the Compound (II). The reaction temperature is 50°C to 80°C, preferably 55°C to 65°C. Then, the Compound (II) is treated with ammonia water to produce the Compound (Ia). Also, the Compound (II) may not be isolated to be subjected to a work-up, then the resulting solution is concentrated, and treated with ammonia water to produce the Compound (Ia).

### (2-2) Production of Compound (A)

The above produced Compound (Ia) is subjected to the above step (1) to produce the target Compound (A).

(3) The Compound (IV) is treated with trifluoroacetic anhydride to produce the Compound (III), and then subjected to the above step (2) to produce the target Compound (A).

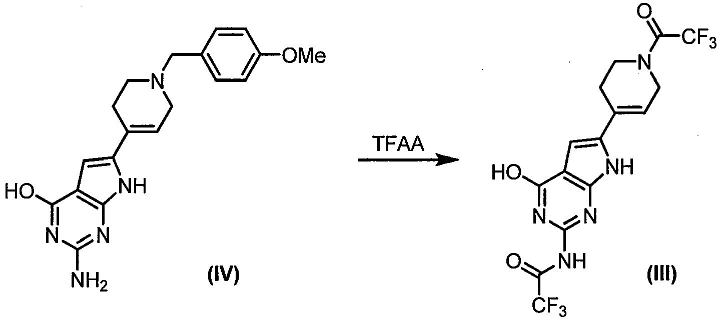

### (3-1) Production of Compound (III)

In acetonitrile or 1,2-dichloroethane, preferably in acetonitrile, in the presence of an amine compound, the Compound (IV) is reacted with trifluoroacetic anhydride. The amine compound is selected from triethylamine, pyridine, and 2,6-lutidine, and 2,6-lutidine is preferable. It is preferable that 6 equivalents or more of amine compound is added, and 5 equivalents or more of trifluoroacetic anhydride is added, relative to the Compound (IV). The reaction temperature is 30°C to 60°C, preferably 35°C to 45°C.

The Compound (III) can be easily isolated, and it is preferable to use the isolated solids in the next step.

### (3-2) Production of Compound (A)

The above produced Compound (III) is subjected to the above step (2) to produce the target Compound (A).

(4) The Compound (V) is treated with sodium borohydride to produce the Compound (IV), and then subjected to the above step (3) to produce the target Compound (A).

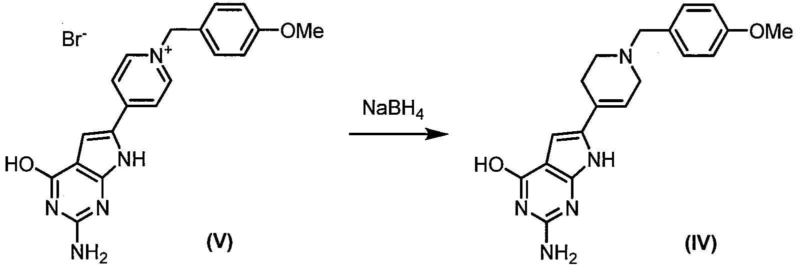

### (4-1) Production of Compound (IV)

The Compound (V) is reacted with 3 to 6 equivalents, preferably 4 equivalents of sodium borohydride. As the solvent, methanol, ethanol, DMA, pyridine, or water may be used, and any one of them may be used or two or more of them may be mixed and used. Among them, a mixture of methanol and pyridine is optimal, and in that case, the ratio of pyridine is preferably 25% to 75%. The reaction temperature is 40°C to 80°C, preferably 50°C to 70°C.

The product Compound (IV) may be isolated as free base or hydrochloride, and preferably isolated as hydrochloride.

### (4-2) Production of Compound (A)

The above produced Compound (IV) is subjected to the above step (3) to produce the target Compound (A).

(5) The Compound (VII) is reacted with a brominating agent to produce the brominated compound (X), and then reacted with diaminopyrimidine (VI) to produce the Compound (V). 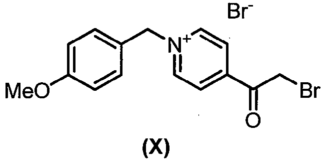 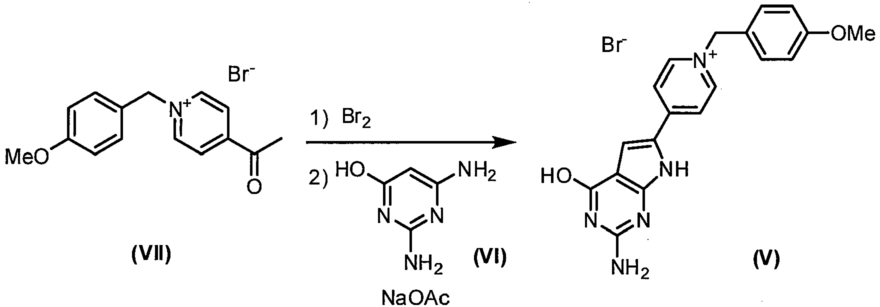 Then, the Compound (V) is subjected to the above step (4) to produce the target Compound (A).

### (5-1) Production of Compound (V)

The Compound (VII) is brominated with bromine or NBS. When NBS is used, a solution of hydrogen bromide in acetic acid is added. The solvent is selected from acetic acid, dichloromethane, and chloroform. The reaction temperature is preferably 0°C to 30°C. The resulting brominated compound (X) may be isolated, or directly used in the next reaction as a reaction mixture.

The reaction of the brominated compound (X) and diaminopyrimidine (VI) is carried out in acetic acid or a mixture of acetic acid and water. When a mixture of acetic acid and water is used, the ratio of water is preferably 1% to 90%. The reaction temperature is 50°C to 90°C, preferably 70°C to 85°C.

### (5-2) Production of Compound (A)

The above produced Compound (V) is subjected to the above step (4) to produce the target Compound (A).

In this regard, the starting material Compound (VII) in the above (5-1) may be produced from anise alcohol (IX), as shown in, for example, the following scheme. 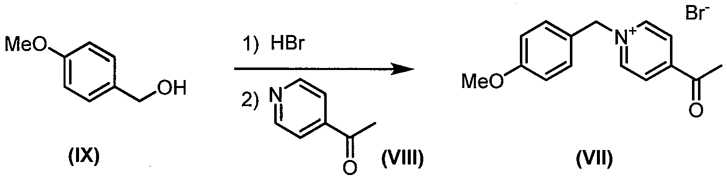 Namely, anise alcohol (IX) is reacted with hydrogen bromide to produce 4-methoxybenzyl bromide, and then reacted with the Compound (VIII).

The reaction of anise alcohol (IX) and hydrogen bromide is carried out in a solvent selected from toluene, dichloromethane, and chloroform, and hydrogen bromide is added as an aqueous solution. The reaction temperature is 0°C to 50°C, more preferably 15°C to 30°C. The product 4-methoxybenzyl bromide may be isolated once and then used in the next reaction, or may be used as a solution in the reaction solvent. The subsequent reaction with the Compound (VIII) is carried out in a solvent selected from toluene, dichloromethane, and chloroform. The reaction temperature is 0°C to 50°C, preferably 15°C to 30°C. The product Compound (VII) can be easily isolated as solids, and it is preferable to isolate said compound and then use it in the next step.

A compound represented by the following formula (Ia): 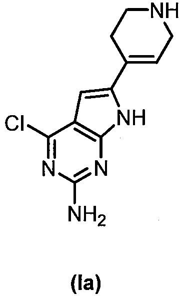 or a salt thereof is a production intermediate for producing the Compound (A), and encompassed by the present invention. The followings are embodiments of the invention for producing said production intermediate.

(6) The Compound (III) is chlorinated, and then the trifluoroacetyl group is deprotected to produce the production intermediate (Ia).

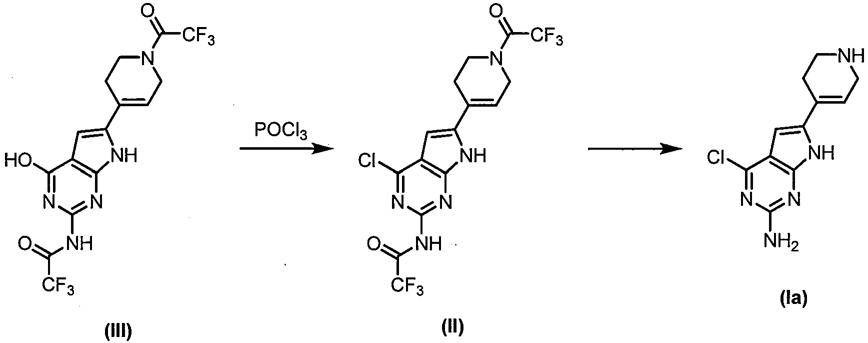

The productions of the Compound (II) and (Ia) are as described in the above step (2-1).

(7) The Compound (IV) is treated with trifluoroacetic anhydride to produce the Compound (III), and then subjected to the above step (6) to produce the production intermediate (Ia).

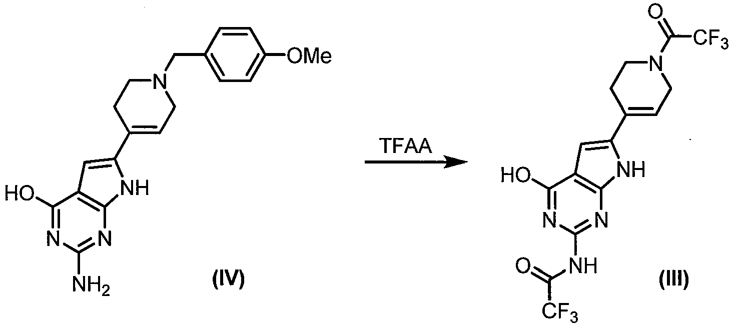

The production of the Compound (III) is as described in the above step (3-1).

(8) The Compound (V) is treated with sodium borohydride to produce the Compound (IV), and then subjected to the above step (7) to produce the production intermediate (Ia).

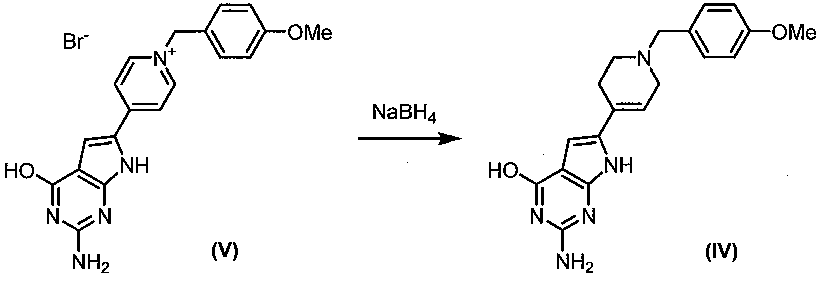

The production of the Compound (IV) is as described in the above step (4-1).

(9) The Compound (VII) is reacted with a brominating agent, then reacted with diaminopyrimidine (VI) to produce the Compound (V), and then subjected to the above step (8) to produce the production intermediate (Ia).

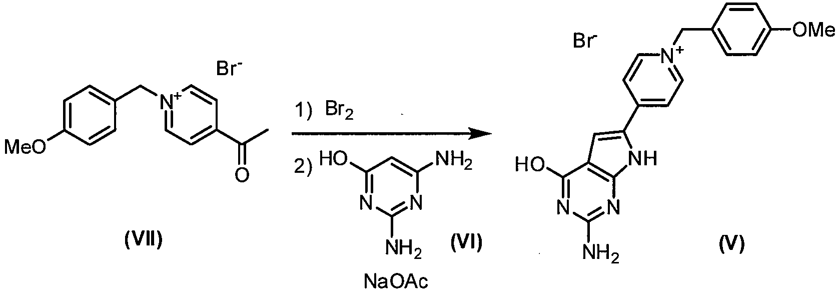

The production of the Compound (V) is as described in the above step (5-1).

Hereinafter, the present invention is specifically illustrated by an Example and a Reference Example, but these Example and Reference Example do not limit the scope of the present invention.

Also, the Compound (A) produced via the production intermediate of the present invention is currently in clinical development. The production method of the Compound (A) is described as a Reference Example.

### EXAMPLES

### Example 1

### Production of 4-chloro-6-(1,2,3,6-tetrahydropyridin-4-yl)-7H-pyrrolo[2,3-d]pyrimidin-2-amine (Ia)

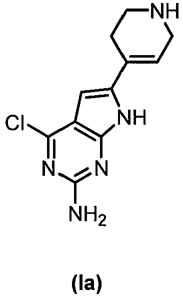

### (Step 1) 4-Acetyl-1-(4-methoxybenzyl)pyridin-1-ium bromide (VII)

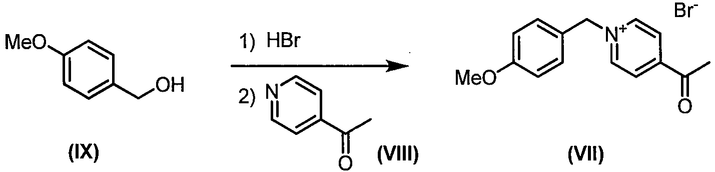

Hydrobromic acid (48wt% in water, 987 mL, 8.8 molar, 3 Eq, 8.68 mol) was added to a mechanically stirred solution of anise alcohol (IX) (400 g, 1 Eq, 2.89 mol) in toluene (2 L). After stirring for 1 hour, the resulting two layers were separated. The water layer was extracted with toluene (2 x 500 mL). The combined organic layers were dried over sodium sulfate, filtered, and the solids were washed with toluene (400 mL). 1-(Pyridin-4-yl)ethan-1-one (VIII) (351 g, 1 Eq, 2.89 mol) was added thereto in one portion. The reaction mixture was heated at 60°C for 23 hours. The solids were isolated by suction filtration, washed with toluene, and dried under reduced pressure to yield 133.13 g of product. The big lump of solids sticking to the bottom of the flask was removed and broken into smaller pieces, which yielded a second batch of 730.5 g. Total yield: 863.63 g (93%).
¹H NMR (400 MHz, DMSO-*d*₆) δ 9.44 - 9.36 (m, 2H), 8.48 (d, *J* = 6.1 Hz, 2H), 7.62 - 7.50 (m, 2H), 7.07 - 6.96 (m, 2H), 5.89 (d, *J* = 5.4 Hz, 2H), 3.75 (s, 3H), 2.72 (s, 3H).LCMS (m/z): 242.4 [M]⁺

### (Step 2) 4-(2-Amino-4-hydroxy-7H-pyrrolo[2,3-d]pyrimidin-6-yl)-1-(4-methoxybenzyl)pyridin-1-ium bromide (V)

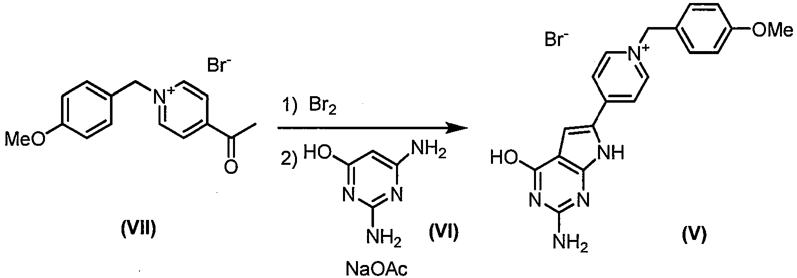

4-Acetyl-1-(4-methoxybenzyl)pyridin-1-ium bromide (VII) (617 g, 1.0 Eq, 1.91 mol) was dissolved in acetic acid (1.25 L) in a 20 L flask. A solution of bromine (306 g, 98.7 mL, 1.0 Eq, 1.91 mol) in acetic acid (625 mL) was added thereto by means of a dropping funnel over a 10 minutes period. After stirring for 55 minutes, 2,6-diaminopyrimidin-4-ol (VI) (242 g, 1.0 Eq, 1.91 mol) was added thereto in one portion, followed by a solution of sodium acetate trihydrate (521 g, 2.0 Eq, 3.83 mol) in water.

After stirring for 1 hour 45 minutes at room temperature and additional stirring for 3 hours 30 minutes at 80°C, heating was stopped and the mixture was stirred overnight at room temperature. The reaction mixture was filtered over a P2 glass filter under suction to give pale orange wet solids and orange filtrate. The orange solids on the glass filter were washed with water (3 x 500 mL) and then dried under reduced pressure to yield the title compound as orange solids. Yield: 637.42 g (78%).
¹H NMR (400 MHz, DMSO-*d*₆) δ 12.19 (s, 1H), 10.73 (s, 1H), 8.81 (d, *J =* 6.9 Hz, 2H), 8.14 (d, *J* = 6.8 Hz, 2H), 7.68 (s, 1H), 7.56 - 7.45 (m, 2H), 7.03 - 6.94 (m, 2H), 6.74 (s, 2H), 5.52 (s, 2H), 3.75 (s, 3H). LCMS (m/z): 348.4 [M]⁺

### (Step 3) 2-Amino-6-(1-(4-methoxybenzyl)-1,2,3,6-tetrahydropyridin-4-yl)-7H-pyrrolo[2,3-d]pyrimidin-4-ol (IV)

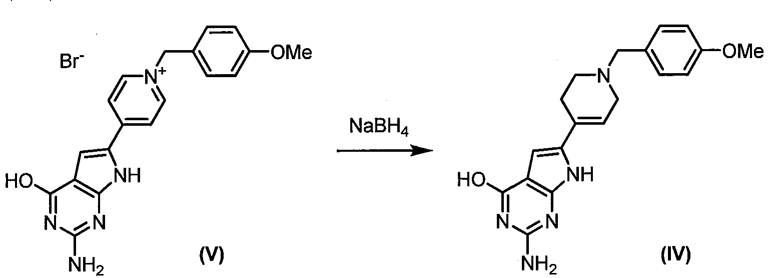

At room temperature, sodium borohydride (223.5 g, 3.0 Eq, 5.9081 mol) was added in portions to a stirred suspension of 4-(2-amino-4-hydroxy-7H-pyrrolo[2,3-d]pyrimidin-6-yl)-1-(4-methoxybenzyl)pyridin-1-ium bromide (V) (843.46 g, 1.0 Eq, 1.96 mol) in a mixed solvent of methanol (1.675 L) and pyridine (5.025 L). After heating for 4 hours 15 minutes with keeping the internal temperature around 40°C, the reaction mixture was cooled in an ice-water bath. Water (5 L) was added thereto, followed by the slow addition of concentrated hydrochloric acid (1 L) thereto with keeping the temperature at 28°C or below. The solids were isolated by suction filtration over a Büchner funnel with 2 layers of filter paper. The resulting yellow solids were washed with water (2 x 2 L), acetonitrile (1 L), and then TBME (2 x 1 L). After drying overnight under a stream on nitrogen, the isolated material was dried under reduced pressure at 50°C to yield the title compound. Yield: 628.4 g (91%).
¹H NMR (400 MHz, DMSO-*d*₆) δ11.31 (d, *J* = 2.4 Hz, 1H), 10.37 (d, *J* = 12.3 Hz, 1H), 7.53 (td, *J* = 8.5, 2.6 Hz, 2H), 7.02 (dd, *J* = 8.8, 2.5 Hz, 2H), 6.33 - 6.23 (m, 3H), 6.02 (d, *J* = 4.4 Hz, 1H), 4.37 - 4.24 (m, 2H), 3.79 (d, *J =* 0.8 Hz, 3H), 3.70 (s, 2H), 3.51 (s, 1H), 3.12 (d, *J =* 10.1 Hz, 1H), 2.70 (s, 2H).
LCMS (m/z): 352.0 [M+H]⁺

### (Step 4) 2,2,2-Trifluoro-N-(4-hydroxy-6-(1-(2,2,2-trifluoroacetyl)-1,2,3,6-tetrahydropyridin-4-yl)-7H-pyrrolo[2,3-d]pyrimidin-2-yl)acetamide (III)

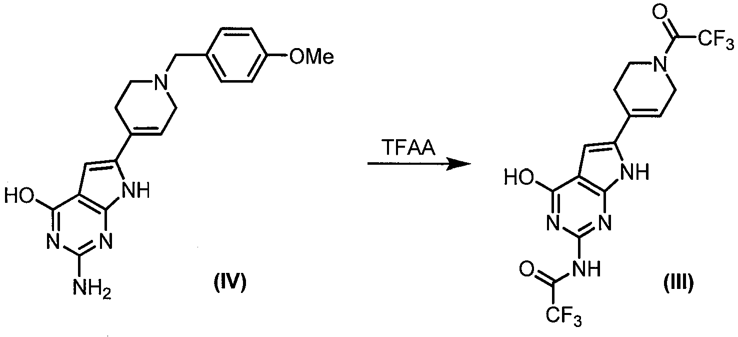

Trifluoroacetic anhydride (598 g, 402 mL, 5 Eq, 2.85 mol) was added slowly by means of a dropping funnel to a stirred suspension of 2-amino-6-[1-(4-methoxybenzyl)-1,2,3,6-tetrahydropyridin-4-yl]-7*H*-pyrrolo[2,3-*d*]pyrimidin-4-ol (IV) (200 g, 1.0 Eq, 569 mmol) and 2,6-lutidine (467 g, 505 mL, 7.66 Eq, 4.36 mol) in acetonitrile (1.0 L), with keeping the internal temperature below 35°C. After 35 minutes, the addition was completed and the cooling bath was removed. After stirring for additional 3 hours, the reaction mixture was poured into water (2.5 L) containing ice (6 L). The red/orange solids were isolated by suction filtration over a Büchner funnel with paper. The isolated compound was transferred into a round bottom flask and heated in acetonitrile (around 400 mL) to 70°C, and then cooled in an ice-water bath. The mixture was filtered over a Büchner funnel with paper, and washed with acetonitrile and TBME. The resulting solids were dried under reduced pressure at 50°C at 20 mbar to yield the title compound. Yield: 163.37 g (68%).
¹H NMR (400 MHz, DMSO-*d*₆) δ12.41 (s, 1H), 12.05 (s ,1H), 11.64 (s, 1H), 6.51 (d, *J =* 0.6Hz, 1H), 6.29 (s, 1H), 4.26 (d, *J =* 10.5 Hz, 2H), 3.75 (s, 2H), 2.57 (s, 2H).
LCMS (m/z): 424.2 [M+H]⁺

### (Step 5) N-(4-chloro-6-(1-(2,2,2-trifluoroacetyl)-1,2,3,6-tetrahydropyridin-4-yl)-7H-pyrrolo[2,3-d]pyrimidin-2-yl)-2,2,2-trifluoroacetamide (II)

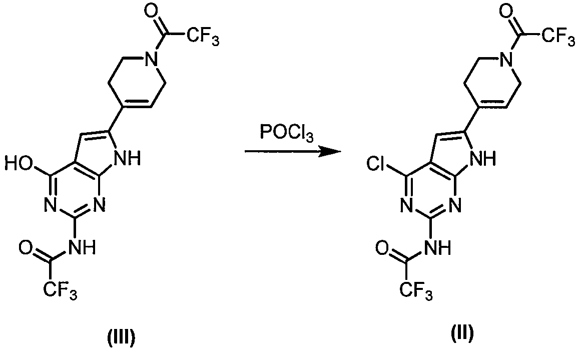

In a 3-necked round-bottom flask equipped with a mechanic stirrer and reflux condenser, a mixture of 2,2,2-trifluoro-*N*-{4-hydroxy-6-[1-(2,2,2-trifluoroacetyl)-1,2,3,6-tetrahydropyridin-4-yl]-7*H*-pyrrolo[2,3-*d*]pyrimidin-2-yl}acetamide (III) (163.3 g, 1 Eq, 385.8 mmol), acetonitrile (500 mL), and phosphorus oxychloride (354.9 g, 215.7 mL, 6 Eq, 2.315 mol) was heated to reflux. After heating overnight, the resulting brown solution was cooled in an ice-water bath. The crude reaction mixture was directly poured onto water (1.8 L) containing ice (3.0 L) with vigorous manual stirring for 5 minutes. Then, stirring was continued mechanically for 30 minutes. The resulting solids were isolated by suction filtration over a Büchner funnel and washed with water (3 x 250 mL). The resulting yellow solids were co-evaporated with acetonitrile (3 x 250 mL) under reduced pressure and dried further under reduced pressure at 50°C. Yield: 87.9 g (52%)
¹H NMR (400 MHz, DMSO-*d*₆) δ12.88 (d, *J* = 4.1 Hz, 1H), 12.19 (s, 1H), 6.62 (s, 1H), 6.59 (d, *J* = 1.2 Hz, 1H), 4.30 (m, 2H), 3.80 (m, 2H), 2.66 (m, 2H).
LCMS (m/z): 442.2 [M+H]⁺

### (Step 6) 4-Chloro-6-(1,2,3,6-tetrahydropyridin-4-yl)-7H-pyrrolo[2,3-d]pyrimidin-2-amine (Ia)

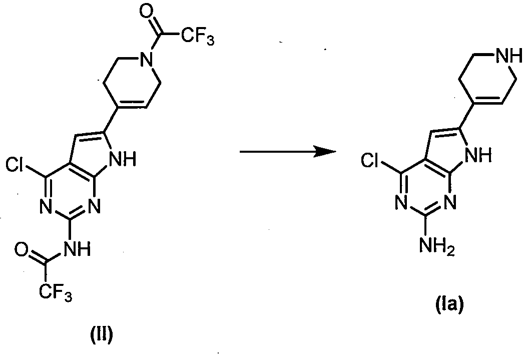

A suspension of N-{4-chloro-6-[1-(2,2,2-trifluoroacetyl)-1,2,3,6-tetrahydropyridin-4-yl]-7H-pyrrolo[2,3-*d*]pyrimidin-2-yl}-2,2,2-trifluoroacetamide (II) (50.0 g, 1 Eq, 113 mmol) in 2-propanol (200 mL) was stirred at room temperature. After almost all solids had dissolved, ammonia water (154 g, 196 mL, 25.0% Wt, 20 Eq, 2.26 mol) was added thereto. After stirring overnight at room temperature, the resulting white solids were isolated by suction filtration over P3-filter, washed with acetonitrile followed by diethyl ether, and transferred with acetonitrile into a 250 mL round-bottom flask. The resulting mixture was concentrated under reduced pressure at 40 to 50°C. Yield: 26.89 g (95%).
¹H NMR (400 MHz, DMSO-*d*₆) δ 11.76 (s, 1H), 6.60 (s, 2H), 6.35 (s, 1H), 6.28 (s, 1H), 3.68 (s, 2H), 3.20 (s, 2H), 2.60 (s, 2H).
LCMS (m/z): 250.2 [M+H]⁺

### Reference Example 1

### Production of 2-(3-{2-amino-6-[1-(oxetan-3-yl)-1,2,3,6-tetrahydropyridin-4-yl]-7H-pyrrolo[2,3-d]pyrimidin-4-yl}-2-(hydroxymethyl)phenyl)-6-cyclopropyl-8-fluoroisoquinolin-1(2H)-one (Compound A)

### (1) Production of 4-chloro-6-[1-(oxetan-3-yl)-1,2,3,6-tetrahydropyridin-4-yl]-7H-pyrrolo[2,3-d]pyrimidin-2-amine (Ib)

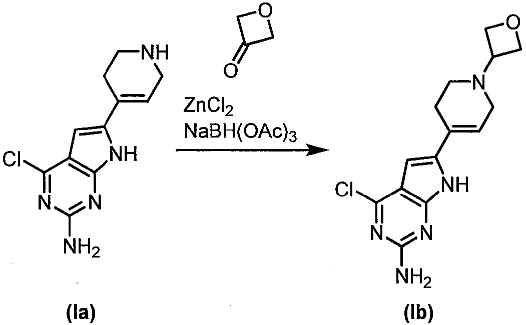

In DMF (128 mL, 8.5 vol.), the Compound (Ia) (15.00 g, 1.0 eq) was stirred, 3-oxetanone (8.66 g, 2.0 eq) was added thereto under nitrogen atmosphere, and then zinc chloride (0.82 g, 0.10 eq) was added thereto. The resulting mixture was stirred at 20°C for 1 hour. Sodium triacetoxyborohydride (30.56 g, 2.4 eq) was added thereto in three portions over 90 minutes, and then the resulting mixture was stirred at 20°C for 1 hour. With keeping the internal temperature at 10°C or below, water (75 mL, 5 vol.) and 20% ammonia water (30 mL, 2 vol.) were added thereto, and additional water (118 mL, 7.9 vol.) was added thereto. The resulting mixture was warmed to 20°C, then cooled to 5°C again, and the precipitated product was collected by filtration. The resulting filter cake was washed with water (45 mL x 3) and 2-propanol (45 mL, 3 vol.), and dried at 55°C overnight to give the Compound (Ib). The resulting residue was cooled to 5°C (internal temperature), and water was added thereto to produce heat and gas, while the internal temperature was kept at 10°C or below. Additional water was added thereto at 10°C or below to confirm that the pH was 11 to 12. The resulting mixture was stirred at 5 to 20°C for 14 to 15 hours, and then the product was collected by filtration. The resulting solids were washed with water until the mother liquor became neutral, and then washed with 2-propanol. The solids were dried at 55°C overnight to give the Compound (Ib) as a crude product. Yield: 14.7 g (80%)
¹H NMR (400 MHz, DMSO-*d*₆) δ 11.58 (s, 1H), 6.53 (s, 2H), 6.33 (s, 1H), 6.18 (s, 1H), 4.53 (dt, *J* = 12.2, 6.3 Hz, 4H), 3.53 (p, *J* = 6.3 Hz, 1H), 2.99 (s, 2H), 2.46 (d, *J* = 3.8 Hz, 4H).
LCMS (m/z): 306.8 [M+H]⁺

### (2) Production of Compound A

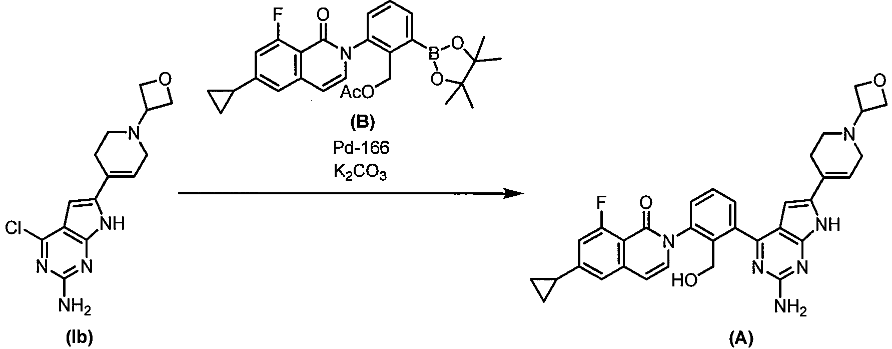

In a solvent of DMSO-water (8:3), the Compound (Ib) (15.0 g, 1.0 eq), the Compound (B) (25.76 g, 1.1 eq), and potassium carbonate (13.56 g, 2.0 eq) were added thereto, and Pd-166 (0.27 g, 0.8% mol) was additionally added thereto under nitrogen atmosphere. The reaction mixture was heated at 100°C (internal temperature) for about 40 minutes, and then stirred at the same temperature for 3 hours or more. The mixture was cooled to 90°C over 15 minutes or more, and an equal amount of water to DMSO was added thereto over 1.5 hours or more. The resulting mixture was stirred at 90°C for at least 1 hour, and then stirred at 20°C (internal temperature) for 2 hours or more. The product was collected by filtration, washed with DMSO-water (1.5:3), and washed with water. The product was dried at 60°C under reduced pressure to give the target compound. Yield: 29.5 g (97%)
¹H NMR (400 MHz, DMSO-*d*₆) δ 11.52 (s, 1H), 7.72 (dd, *J =* 7.7, 1.1 Hz, 1H), 7.59 (t, *J =* 7.8 Hz, 1H), 7.46 (dd, *J* = 7.8, 1.1 Hz, 1H), 7.36 (d, *J* = 7.4 Hz, 1H), 7.27 (d, *J* = 1.3Hz, 1H), 6.99 (dd, *J =* 13.2, 1.3 Hz, 1H), 6.62 (dd, *J* = 7.5, 2.0 Hz, 1H), 6.41 (s, 2H), 6.35 (s, 1H), 6.21 (s, 1H), 5.18 (dd, *J* = 8.8, 4.5 Hz, 1H), 4.53 (dt, *J* = 30.5, 6.3 Hz, 4H), 4.25 (dd, *J =* 12.0, 4.4 Hz, 1H), 4.05 (dd, *J* = 11.9, 9.0 Hz, 1H), 3.54 (m, 4H), 3.01 (s, 2H), 2.46 (d, *J =* 4.0 Hz, 4H), 2.20-2.14 (m, 1H), 1.14-1.05 (m, 2H), 0.93-0.80 (m, 2H).

### INDUSTRIAL APPLICABILITY

The compound of the present invention is useful as a production intermediate of the Compound (A) having a BTK inhibitory action.

By using said intermediate, the Compound (A) can be produced more economically and in higher yields.

## Claims

1. A compound represented by the following formula (Ia) 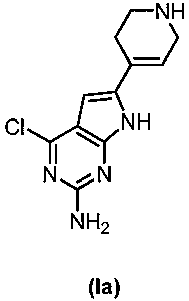 or a salt thereof.
